Europäisches Patentamt

European Patent Office

Office européen des brevets

(11) Publication number: **0 434 147 A2**

# EUROPEAN PATENT APPLICATION

(21) Application number: 90203322.4

(51) Int. Cl.5: **B01L 3/14**, A61B 5/14

(22) Date of filing: 13.12.90

(30) Priority: 22.12.89 US 455485

(43) Date of publication of application:
26.06.91 Bulletin 91/26

(84) Designated Contracting States:
BE CH DE FR GB IT LI LU NL

(71) Applicant: EASTMAN KODAK COMPANY
Patent Department, 343 State Street
Rochester, NY 14650-2201(US)

(72) Inventor: Columbus, Richard Lewis, c/o
EASTMAN KODAK COMPANY
Patent Department, 343 State Street
Rochester, New York 14650(US)

(74) Representative: Phillips, Margaret Dawn et al
Kodak Limited Patent Department Headstone
Drive
Harrow, Middlesex HA1 4TY(GB)

(54) Kit of collection vessels of uniform outside dimensions, different volumes.

(57) Phlebotomy syringes are designed to receive tubes of a predetermined fixed length and external diameter. However, if the tube designed to fit the syringe is of a particular size which has a large volume, it is often difficult to remove a low volume sample collected therein. Described herein is a kit of collection vessels (20a, 20b, 20c, 20d). Each collection vessel (20a, 20b, 20c, 20d) in the kit has the same overall length (L) and outside diameter (D) to allow interface with a single other device, for example a phlebotomy syringe. A partition (30) is used to close off one part of the tube and in some of the tubes, it is positioned to create differing volumes which are less than the total possible volume.

V=7.0ml    V'=2.5ml    V''=4.5ml    V'''=3.5ml

FIG. 2

Xerox Copy Centre

## KIT OF COLLECTION VESSELS OF UNIFORM OUTSIDE DIMENSIONS, DIFFERENT VOLUMES

The invention relates to a kit of collection vessels, particularly those useful as phlebotomy tubes which cooperate with a syringe.

In the field of phlebotomy, tubes are used to receive blood from a needle, such as those partially evacuated for automatic blood intake. Kits of such tubes ace disclosed in US-A-3405706. However, the tubes shown in US-A-3405706 all have the same volume. It is conventional to provide tubes of differing volume, since in some instances much less blood is needed, e.g. for pediatrics. For a large volume tube, the small contained blood volumes are difficult to remove after collection due to the large surface area. Prior to this invention, such tubes of differing volume have also necessitated different exterior sizes, e.g. the smaller volume tubes have also been shorter and/or narrower than the larger volume tubes.

Such difference in overall size as well as volume is advantageous in identifying (by shape) which tube is for what volume. However, it has a substantial disadvantage. In use, the tubes are inserted into a phlebotomy syringe, and in most instances the syringe is designed to receive a tube having a predetermined fixed length and diameter. For example, the phlebotomy tube described in US-A-4813426 snugly fits the diameter of the syringe which holds it. That particular syringe will not accommodate a tube of larger diameter, and will only awkwardly receive one of smaller diameter due to the sloppy fit which ensues. The sloppy fit which occurs in such a case is particularly disadvantageous when drawing blood from a child who may be very active and who may disturb the loose-fitting tube.

Hence, differently-sized collection tubes normally necessitate differently-sized syringes, which in turn leads to added expense and procedures.

Therefore, prior to this invention, a problem has been to provide a kit of universally sized blood collection tubes to be used in a phlebotomy syringe, which will allow differing volumes to be collected in such tubes, all with the same single phlebotomy syringe.

The above problems are addressed by a kit of phlebotomy tubes which avoids the above-noted problems.

More specifically, in accordance with one aspect of the present invention, there is provided a kit of liquid collection vessels, each vessel having the same overall dimensions of length, width and thickness, but different predetermined volumes, each vessel comprising a container having opposed ends, at least one end of which is adapted to receive a liquid, and a solid partition is provided to close off liquid flow away from that end of the container,

characterized in that the partition in at least one of the vessels is positioned at a location partway between the ends, and is selected to divide the volume of that particular vessel into two parts, each part forming one of the predetermined volumes.

It is an advantageous feature of the invention that devices which must interact with the vessels of the invention, such as a phlebotomy syringe, need only be a single size, since the uniform sized vessels of the kit will nevertheless accommodate different volumes.

It is a related advantageous feature of the invention that a kit of collection vessels is provided wherein all the vessels are of uniform exterior size, and approximately uniform surface/volume ratio, but each with a markedly different possible volume.

The present invention will now be described by way of example only with reference to the accompanying drawing in which:

Figure 1 is a perspective view of a kit of collection vessels in accordance with the present invention; and

Figure 2 is a sectioned elevational view of the vessels of the kit shown in Figure 1.

The invention is hereinafter described by reference to the preferred embodiments, in which the collection vessels are phlebotomy tubes for collecting whole blood. In addition, the invention is useful regardless of the liquid being collected, and, is thus not limited just to phlebotomy tubes. The invention is useful wherever there is an advantage to be gained from a universal size but different volumes, such as because the collection vessel must interface with another device which needs to be fitted to the vessel.

It is evident that certain fluid analysis programs or test schemes can require more vessels of one size than of another, and in fact, that some schemes may exclude use of certain sizes altogether. It is thus understood that "kit" means not merely a holder with an array of tubes, but a supply of tubes, with or without a holder, the supply providing different sized vessels, with or without individual wraps, to allow renewal of the most used sizes, the characterization of the kit being that all its member vessels are of the same general length, width and thickness, with a common means, such as a septum, to adapt the vessel for fluid withdrawal or dispensing.

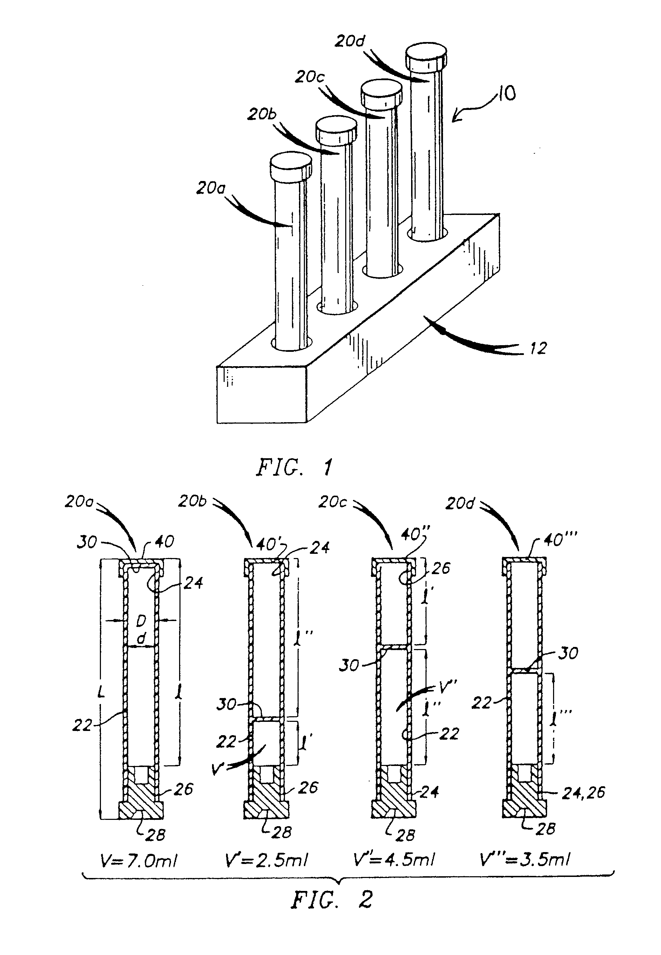

As shown in Figure 1, kit 10 preferably comprises a suitable package which holds all of the phlebotomy tubes 20a, 20b, 20c and 20d together. In addition to the bottom holder 12 of the package which is shown, there can be a suitable top (not shown) which is hinged or removed from holder 12.

The basic tube is that shown as tube 20a. As is shown more clearly in Figure 2, tube 20a preferably comprises a cylinder which is preferably formed from a molded plastic. The cylinder has sidewalls 22, having opposite ends 24 and 26. End 26 is open, and is constructed to receive a puncturable septum 28, as is conventional. Sidewalls 22 extend for a length L with an outside diameter D.

To prevent liquid flow away from end 26 out of tube 20a, e.g., at opposite end 24, a solid partition 30 is formed, preferably integral with sidewalls 22. In the case of tube 20a, partition 30 is at the end 24, enabling tube 20a to collect blood over the longest possible length $\ell$. The volume in such a case can be, e.g. 7ml, which is usually the largest volume used in phlebotomy devices.

On the other hand, tubes 20b, 20c and 20d each have lesser volumes of collection, although they are of the same cylindrical shape with the same sidewalls 22 having the same overall length L and overall diameter D. The useful volumes of these tubes (and blood collection length $\ell'$ $\ell''$ and $\ell'''$ respectively) are reduced by the location of the partition 30. In tube 20b, partition 30 is located so that $\ell'$ and $\ell''$, as well as the useful volumes of the two parts so formed, are in a ratio of preferably 5 to 9. For example, the volumes become about 2.5 to 4.5ml from the original 7.0ml volume of tube 20a. Tube 20c is substantially similar to tube 20b, except that septum 26 is in end 24 instead of end 26.

Of the ends of tubes 20b and 20c, the end which is opposite to the septum end (24 and 26 respectively) is capped with a color-coded cap 40' or 40" which serves as a visual indicia of the volume of that tube. A similar cap 40 is used on tube 20a, being yet a different color in this instance. Alternatively, a raised number (not shown) can be formed in the cap to indicate its volume.

Tube 20d is also generally identical to the other tubes, except that partition 30 is formed halfway along the length L of sidewalls 22, leaving about half the usable volume for blood collection. Septum 28 is positioned in either end 24 or 26. Tube 20d also has its own color - or otherwise-coded cap 40'" positioned at the end opposite to the end with the septum.

Of these tubes, preferably tube 20b with volume V' is the pediatric (or geriatric) tube, as it uses the least volume of blood.

By this invention, both the volume and surface area increase generally in proportion to the increased value of useful collection length $\ell$. Thus, when length $\ell'$ is used, approximately the same surface-to-volume ratio is achieved in tube 20b as occurs in tube 20a (the constant surface area of partition 30 being small compared to the surface area of the blood collection cylinder formed $\pi d \cdot \ell$ - (or $\pi d \cdot \ell'$), where d is slightly less than D). The result is that blood in tube 20b can be poured out after collection with the same ease as in any of the other tubes 20a, 20c or 20d. It is only if the volume V' were to be collected in a tube 20a (not done with this invention), that pour-off becomes difficult, due to the larger surface area ($\pi d \cdot \ell$) over which the reduced volume V' is distributed.

Yet other volumes V' and V" can be achieved to give a ratio other than 5/9, by the simple relocation of partition 30 at some other appropriate point along length L.

Any plastic can be used for cylinder 22 which will hold a vacuum for the desired storage length. The following plastics have been found to be superior for this purpose: polyethylene terephthalate, or any of the polymers described in Research Disclosure, published by Emsworth Studios, 260 West 39 Street, New York, NY 10018, Nos. 29416 and 29484, in the October, 1988 issue. More specifically, the latter include blends of poly(ethylene 2,6-naphthalenedicarboxylate) or its copolyester, with from 1 to 30 weight percent of poly(ethylene-co-vinyl alcohol). These blends demonstrate a low degree of gas permeability, so that the blends are unusually effective in holding partial vacuum during storage.

Alternatively, if the kit is to be used for other than evacuated containers, almost any plastic will suffice which is inert to the liquid to be collected.

Any of the tubes 20a to 20d or the septum 28, or both, can be provided with flats (not shown) to prevent the tubes from rolling on a flat surface.

Additionally, any of the tubes can have added to the walls prior to use, addenda useful in handling the blood, for example, ethylenediaminetetraacetic acid (EDTA) and the like.

In yet another form of the invention (not shown), a septum is included in both of the opposite ends 24 and 26 of tubes 20b, 20c or 20d, whereby both volumes can be used. The septum is then color-coded or otherwise marked for its volume V', V" or V'".

## Claims

1. A kit of liquid collection vessels (20a, 20b, 20c, 20d), each vessel (20a, 20b, 20c, 20d) having the same overall dimensions of length (L), width and thickness, but different predetermined volumes (V, V', V", V'"), each vessel (20a, 20b, 20c, 20d) comprising a container (22) having opposed ends (24, 26), at least one end of which is adapted to receive a liquid, and a solid partition (30) is provided to close off liquid flow away from that end of the container (22),

characterized in that the partition (30) in at least one of the vessels (20b, 20c, 20d) is positioned at a location partway between the ends (24, 26), and is selected to divide the volume of that particular vessel (20b, 20c, 20d) into two parts, each part forming one of the predetermined volumes (V', V", V'").

2. A kit according to claim 1, wherein the parts are unequal in volume.

3. A kit according to claim 1 or 2, wherein each vessel (20a, 20b, 20c, 20d) bears an indicia of the volume (V, V', V", V'") contained therein.

4. A kit according to any one of the preceding claims, wherein the parts have individual volumes which form a volume ratio of about 5/9.

5. A kit according to any one of the preceding claims, wherein the liquid collection vessels are phlebotomy tubes.

6. A kit according to any one of the preceding claims, wherein the container (22) and the partition (30) comprise a plastic selected from the group consisting of polyethylene terephthalate and a blend of poly(ethylene 2,6-naphthalenedicarboxylate) with from 1 to 30 weight percent of poly(ethylene-co-vinyl alcohol).

FIG. 1

$V=7.0ml$     $V'=2.5ml$     $V''=4.5ml$     $V'''=3.5ml$

FIG. 2